# EUROPÄISCHE PATENTSCHRIFT

(11) **EP 1 449 543 B1**
(45) Veröffentlichungstag und Bekanntmachung des Hinweises auf die Patenterteilung: **26.10.2005**
(21) Anmeldenummer: 04003389.6
(22) Anmeldetag: 16.02.2004
(51) Int. Cl.: A61L 2/18, B08B 3/04, B65B 55/10

(54) **Vorrichtung und Verfahren zur Behandlung von kleineren Gegenständen**
Device and method for treating small objects
Dispositif et méthode pour le traitement de petits objets

(30) Priorität: 20.02.2003 DE 10307444
(43) Veröffentlichungstag der Anmeldung: 25.08.2004
(73) Patentinhaber: Atec Pharmatechnik Gmbh, 24966 Sörup (DE)
(72) Erfinder: Mumm, Hans-Werner, 24966 Sörup (DE)
(74) Vertreter: Thomas, Götz

(56) Entgegenhaltungen:
- DE-U- 29 923 723
- US-A- 3 992 148
- US-A- 5 056 948

## Beschreibung

Die Erfindung betrifft eine Vorrichtung zur Behandlung von kleineren Gegenständen, insbesondere zur Reinigung und Sterilisation von Spritzenkolben oder Verschlüssen von Spritzen, Infusionsflaschen, Vials für pharmazeutische Zwecke, mit einem Behandlungs- und Transportbehälter zur Aufnahme einer Mehrzahl der Gegenstände, der an einem motorisch schwenkbaren Tragelement der Vorrichtung lösbar befestigbar ist und zwei verschließbare Öffnungen aufweist, die nach der Befestigung des Behandlungs- und Transportbehälters am Tragelement beiderseits von einer horizontaler Schwenkachse desselben angeordnet und jeweils mit einer entlang der Schwenkachse durch das Tragelement verlaufenden Zufuhr- bzw. Abfuhrleitung für ein flüssiges Behandlungsmedium verbindbar sind. Die Erfindung betrifft weiter ein Verfahren zur Behandlung von kleineren Gegenständen, insbesondere zur Reinigung und Sterilisation von Spritzenkolben oder Verschlüssen von Spritzen, Infusionsflaschen, Vials für pharmazeutische Zwecke, bei dem eine Mehrzahl der Gegenstände in einen Behandlungs- und Transportbehälter eingebracht wird, der zwei verschließbare Öffnungen aufweist, bei dem der Behandlungs- und Transportbehälter anschließend lösbar an einem motorisch schwenkbaren Tragelement einer Behandlungsvorrichtung befestigt wird, so dass die Öffnungen beiderseits von einer horizontalen Schwenkachse desselben angeordnet sind, und bei dem die Öffnungen jeweils mit einer entlang der Schwenkachse durch das Tragelement verlaufenden Zufuhr- bzw. Abfuhrleitung für ein flüssiges Behandlungsmedium verbunden werden.

Um bei der Verpackung von pharmazeutischen Produkten in Flaschen, Vials, Spritzen oder dergleichen eine Kontamination der Produkte durch verunreinigte Teile dieser Verpackungsbehälter, zum Beispiel durch Verschlussstopfen oder Spritzenkolben aus Gummi oder Kunststoff, zu vermeiden, müssen diese Teile vor dem Anbringen an den Verpackungsbehältern gründlich gereinigt und sterilisiert und anschließend in sterilem Zustand zur Verpackungsmaschine transportiert werden, ohne dass sie durch den Transport kontaminiert werden.

Da es sich um kleine und verhältnismäßig unempfindliche Gegenstände handelt, erfolgt die Reinigung und Sterilisierung gewöhnlich in einem größeren Behandlungsbehälter einer Reinigungs- und Sterilisationsvorrichtung, in den eine Anzahl der Gegenstände eingebracht und anschließend im Behälter mit heißem Wasser oder einem Wasser-Luft-Gemisch ggf. unter Zusatz von Detergenzien oder anderen Additiven gespült wird, um sämtliche Verunreinigungen oder Fremdkörper, soweit vorhanden, zu beseitigen und mit dem flüssigen Spülmedium aus dem Behälter abzuführen, bevor die Gegenstände anschließend durch Dampfzufuhr in den Behälter sterilisiert werden. Neben der Hauptspülung und der Sterilisation kann die Behandlung der Gegenstände weiter eine Silikonisierung, eine Trocknung und eine abschließende Spülung (final rinse) umfassen. Um eine optimale Zirkulation der Behandlungsmedien im Behälter zu gewährleisten, weist dieser gewöhnlich an seiner Oberseite und an seiner Unterseite zwei einander gegenüberliegenden Öffnungen auf, von denen jeweils eine zur Zufuhr bzw. zur Abfuhr des durch den Behälter zirkulierenden Mediums dient. Eine der beiden Öffnungen bildet zweckmäßig zugleich die Befüllungs- und Entleerungsöffnung des Behälters, durch welche die Gegenstände von oben her eingefüllt und bei umgedrehtem Behälter durch ihre Schwerkraft von selbst nach unten ausgetragen werden.

Um zu verhindern, dass die gereinigten und sterilisierten Gegenstände vor ihrer Anbringung an den Verpackungsbehältern in erneuten Kontakt mit kontaminierenden Stoffen gelangen können, wurde in der DE 44 09 659 C2 bereits vorgeschlagen, den Behandlungsbehälter lösbar an der Reinigungs- und Sterilisationsvorrichtung anzukoppeln, so dass er sich nach einem Verschließen der Öffnungen abnehmen und zugleich als Transportbehälter verwenden lässt. Die gereinigten und sterilisierten Gegenstände bleiben im Behälter eingeschlossen und werden im Behälter zu einer Übergabestation einer Verpackungsmaschine transportiert, an der sich der Behälter ähnlich wie an der Reinigungs- und Sterilisiervorrichtung luftdicht ankoppeln lässt, jedoch in umgedrehtem Zustand, so dass die Gegenstände durch die nach unten weisende Befüllungs- und Entleerungsöffnung aus dem Behälter austreten können.

Aus der DE 299 23 723 U1 sind weiter bereits eine Vorrichtung und ein Verfahren der eingangs genannten Art bekannt, bei denen der Behandlungs- und Transportbehälter an einer Tragvorrichtung einer horizontalen Welle lösbar befestigt und um die Achse der Welle schwenkbar gelagert ist, wobei zwei mit Ventilen an der Ober- und Unterseite des Behandlungs- und Transportbehälters lösbar verbundene Zu- und Abfuhrleitungen für mindestens ein Behandlungsmedium, wie Wasser, Dampf, Druckluft von der Welle getragen werden und entlang der Schwenkachse des Behälters durch einen Träger der Welle verlaufen. Um ein im Behälter befindliches flüssiges Behandlungsmedium, wie beispielsweise Heißwasser oder eine Silikondispersion, aus dem Behälter zu entfernen, wird Druckluft über die an der Oberseite des Behälters mündende Leitung in den Behälter zugeführt und das Medium durch die an der Unterseite des Behälters mündende Leitung abgeführt. Diese Leitung weist jedoch zwischen dem Ventil an der Behälterunterseite und der Welle der Vorrichtung einen aufsteigenden Leitungsabschnitt auf, aus dem sich das flüssige Medium mittels Druckluft nicht vollständig entfernen lässt. Da sich nach Beendigung der Druckluftzufuhr das Medium, und zusammen mit diesem auch schwerere abgereinigte Schmutzpartikel, an der tiefsten Stelle der Leitung zwischen dem Ventil und der Behälteröffnung ansammelt, kann dort die Sterilität und insbesondere die Partikelfreiheit nicht immer mit Sicherheit gewährleistet werden. Außerdem müssen an dieser Stelle zurückbleibende Reste von unsterilem flüssigem Medium durch eine erheblich längere Trocknung beseitigt werden, um zu vermeiden, dass sie beim anschließenden Umdrehen des Behälters nach unten stürzen und zu einer Kontamination oder Verunreinigung der gereinigten und sterilisierten Gegenstände führen. Durch die Trocknung kann jedoch das Problem zurückbleibender Schmutzpartikel nicht gelöst werden. Ein weiterer Nachteil bei der bekannten Vorrichtung besteht darin, dass auch in den Zu- und Abfuhrleitungen Reste von Wasser oder anderem flüssigem Medium zurückbleiben können, da diese Leitungen an der horizontalen Welle entlang geführt sind, die eine beträchtliche Länge aufweist, um die durch die einseitige Aufhängung und das hohe Gewicht des Behälters von gewöhnlich mehr als 4000 N auf die Welle aufgebrachten Momente ohne eine Überlastung der Lager in den Träger einzubringen.

Aus der DE 38 41 930 C2 ist weiter bereits ein Verfahren und eine Vorrichtung zur Reinigung und Sterilisation von kleineren Gegenständen bekannt, bei der ein ortsfester Behälter über eine ortsfeste Medienentleerungsleitung mit einem ortsfesten Ablauf verbunden ist.

Ausgehend hiervon liegt der Erfindung die Aufgabe zugrunde, eine Vorrichtung und ein Verfahren der eingangs genannten Art dahingehend zu verbessern, dass eine Gefährdung der Sterilität und Sauberkeit der Gegenstände im Behandlungs- und Transportbehälter durch im Behälter und/oder in den Zufuhr- bzw. Abfuhrleitungen zurückbleibendes flüssiges Medium oder schwere Schmutzpartikel mit einfachen Mitteln vermieden werden kann.

Diese Aufgabe wird erfindungsgemäß dadurch gelöst, dass die Vorrichtung einen zusätzlichen ortsfesten Entleerungsanschluss für das flüssige Behandlungsmedium aufweist, wobei der Entleerungsanschluss tiefer liegt als diejenige Behälteröffnung, die in einer Medienentleerstellung des Behandlungs- und Transportbehälters nach unten weist, und wobei der Entleerungsanschluss in der Medienentleerstellung lösbar mit der nach unten weisenden Öffnung verbindbar ist.

Mit der erfindungsgemäßen Merkmalskombination lässt sich das während eines Behandlungsschrittes in den Behandlungs- und Transportbehälter zugeführte flüssige Medium allein durch seine Schwerkraft und ohne zusätzliche Maßnahmen, wie Ausblasen, vollständig aus dem Behandlungs- und Transportbehälter entfernen, indem man die nach unten weisende verschlossene Öffnung des Behälters mit dem Entleerungsanschluss verbindet. Nach dem Öffnen eines an der Öffnung vorgesehenen Ventils strömt dann das Behandlungsmedium infolge seiner Schwerkraft vollständig durch die Öffnung aus dem Behälter heraus in den Entleerungsanschluss.

Grundsätzlich kann jedoch auch hier das Abfließen des flüssigen Mediums aus dem Behandlungs- und Transportbehälter beschleunigt werden, indem der Behälter über die in der Medienentleerstellung nach oben weisende, auch mit einem Ventil versehene Öffnung mit Druckluft beaufschlagt wird, um das Wasser mit höherer Geschwindigkeit in den Entleerungsanschluss zu treiben.

Um zu vermeiden, dass die während der Behandlung mit der Öffnung des Behälters verbundene Zufuhr- bzw. Abfuhrleitung abgekuppelt werden muss, um die Öffnung mit dem Entleerungsanschluss zu verbinden, weist diese Leitung vorzugsweise einen Austrittsanschluss auf, der dem Entleerungsanschluss in der Medienentleerstellung des Behälters in geringem Abstand gegenüberliegt, so dass er während des Verschwenkens des Behälters ungehindert am Entleerungsanschluss vorbei bewegt werden kann und zur Entleerung des Behälters ohne großen Aufwand flüssigkeitsdicht mit dem Entleerungsanschluss verbunden werden kann.

Eine weitere bevorzugte Ausgestaltung der Erfindung sieht vor, dass die Zufuhr- bzw. Abfuhrleitung, die in der Medienentleerstellung des Behälters mit dessen nach unten weisender Öffnung verbunden ist, im Bereich ihres Austrittsanschlusses mindestens ein Wegeventil aufweist, mit dem sich nach Bedarf die Verbindung zum Entleerungsanschluss öffnen oder schließen lässt. Vorzugsweise sind zwei Wegeventile vorgesehen, die es gestatten, wahlweise entweder den Austrittsanschluss oder den vom Austrittsanschluss nach oben zum Tragelement führenden Abschnitt der Zufuhr- bzw. Abfuhrleitung zu öffnen bzw. zu verschließen, um den Behälter entweder zur Restentleerung mit der Entleerungsöffnung zu verbinden oder zur Spülung des Behälters mit Behandlungsmedium den Transport des letzteren durch den vom Wegeventil nach oben führenden Abschnitt der Zufuhr- bzw. Abfuhrleitung zu ermöglichen.

Die Verbindung zwischen dem Austrittsanschluss und dem Entleerungsanschluss wird vorzugsweise mittels eines Dichtrings hergestellt, der aus einem gummielastisch verformbaren Material besteht, axial verschiebbar in einen ringförmigen Sitz in einem Anschlussflansch des Entleerungsanschlusses eingesetzt ist und auf seiner vom Austrittsanschluss abgewandten Stirnseite mit Druckluft beaufschlagbar ist, um ihn gas- und flüssigkeitsdicht gegen eine gegenüberliegende Dichtfläche eines Anschlussflansches des Austrittsanschlusses sowie gegen Begrenzungsflächen des Sitzes anzupressen.

Um zu vermeiden, dass sich der Austrittsanschluss in axialer Richtung vom Entleerungsanschluss weg bewegt, wenn der Dichtring von der Druckluft gegen seine Dichtfläche gedrückt wird, kann zweckmäßig vorgesehen werden, dass einer der beiden Anschlussflansche des Entleerungsanschlusses oder des Austrittsanschlusses den jeweils anderen Anschlussflansch so hintergreift, dass sich der Austrittsanschluss beim Verschwenken des Behälters ungehindert am Entleerungsanschluss vorbei bewegen lässt.

Im Bereich der Kupplungsstellen zwischen den beiden Öffnungen des Behälters und der jeweils damit verbindbaren Zufuhr- bzw. Abfuhrleitung können ähnliche gepaarte Anschlussflasche vorgesehen sein, von denen ebenfalls einer mit einem mit Druckluft beaufschlagbaren Dichtring versehen ist und nach dem lösbaren Befestigen des Behälters am Tragelement vom anderen Flansch hintergriffen wird, um die Behälteröffnung gas- und flüssigkeitsdicht mit der Zufuhr- bzw. Abfuhrleitung zu verbinden.

Um sicherzustellen, dass in dem Abschnitt der Zufuhr- bzw. Abfuhrleitung zwischen dem Austrittsanschluss und der in der Medienentleerstellung nach unten weisenden Öffnung des Behälters keinerlei Flüssigkeitsreste zurückbleiben, ist dieser Abschnitt vorzugsweise zum Austrittsanschluss und damit zum Entleerungsanschluss hin geneigt.

Wenn die erfindungsgemäße Vorrichtung zur Reinigung und Sterilisation von Verschlüssen von pharmazeutischen Verpackungsbehältern oder anderen Gegenständen für pharmazeutische Zwecke verwendet werden soll, muss die Reinigung und die Sterilisation der Gegenstände in einer Reinraumumgebung erfolgen. In diesem Fall ist es von Vorzug, das Tragelement mit dem lösbar daran befestigten Behälter sowie Teile der mit den Öffnungen des Behälters verbindbaren Zufuhr- und Abfuhrleitungen in einem Reinraum anzuordnen und durch eine Trennwand vom Rest der Vorrichtung zu trennen, so dass der letztere im Hinblick auf die Sauberkeit und Sterilität nicht dieselben strengen Anforderungen wie die vorgenannten, in den Reinraum ragenden Teile erfüllen muss.

Eine bevorzugte Ausgestaltung der Erfindung sieht dann vor, dass der Entleerungsanschluss in der Trennwand angeordnet ist, in den Reinraum mündet und zweckmäßig auf der vom Reinraum abgewandten Seite durch ein Ventil verschließbar ist, um eine Kontamination des Reinraums durch aus dem Entleerungsanschluss strömende ungereinigte Luft zu vermeiden.

Da auch außerhalb des Reinraums in den Zufuhr- bzw. Abfuhrleitungen zurückbleibendes flüssiges Medium Probleme im Hinblick auf eine Aufrechterhaltung des Reinraumstatus verursachen kann, sieht eine andere bevorzugte Ausgestaltung der Erfindung und für sich selbst patentbegründende Erfindungsvariante vor, dass in der Medienentleerstellung des Behandlungs- und Transportbehälters die beiden als Zufuhr- bzw. Abfuhrleitung dienenden Leitungen nach ihrer Zusammenführung im Bereich des Tragelements bis zu einer verschließbaren Entleerungsöffnung ausschließlich abwärts oder über kurze Strecken horizontal verlaufen, jedoch keinerlei aufsteigende Leitungsabschnitte aufweisen, so dass sie sich in der Medienentleerstellung bei geöffneter Entleerungsöffnung von selbst vollständig entleeren. Vorzugsweise schließen die beiden Leitungen auf der vom Reinraum abgewandten Seite der Trennwand jeweils ein flexibles Leitungsstück ein, das in der Medienentleerstellung des Behandlungs- und Transportbehälters um einen Winkel zwischen 90 und 180 Grad nach unten und in Richtung des Transportbehälters gebogen ist und eine ausreichende Länge aufweist, so dass es beim Verschwenken des Behandlungs- und Transportbehälters um einen Winkel von etwa 130 Grad in beiden Drehrichtungen nicht mit einer Zugspannung beaufschlagt wird und minimalen Torsionsbelastungen ausgesetzt ist.

Um längere horizontale Leitungsabschnitte entlang eines langgestreckten Tragelements zu vermeiden, sieht eine weitere bevorzugte Ausgestaltung der Erfindung und für sich selbst ebenfalls patentbegründende Erfindungsvariante vor, dass das Tragelement im Bereich der Trennwand an einem motorisch antreibbaren Zahnkranz befestigt ist, durch den die Zufuhr- bzw. Abfuhrleitung hindurchgeführt sind. Auf diese Weise müssen die Zufuhr- bzw. Abfuhrleitung nur über eine kurze Strecke von wenigen Dezimeter parallel zur Schwenkachse des Tragelements verlaufen. Zudem weist der Antrieb eine sehr geringe Baulänge auf und gestattet eine ausgezeichnete Lastübertragung des Gewichts des Behandlungs- und Transportbehälters in einen im Bereich der Trennwand angeordneten Maschinenrahmen der Vorrichtung.

Auf seiner dem Behandlungs- und Transportbehälter zugewandten Seite ist der Zahnkranz durch eine Verschlussplatte verschlossen, die mit integrierten Leitungsdurchführungen für die Zufuhr- bzw. Abfuhrleitung sowie für elektrische Leitungen und ggf. Druckluftleitungen versehen ist.

Im folgenden wird die Erfindung anhand eines in der Zeichnung dargestellten Ausführungsbeispiels näher beschrieben. Es zeigen:
Fig. 1: eine teilweise geschnittene schematische Seitenansicht einer erfindungsgemäßen Vorrichtung zur Reinigung und Sterilisation von pharmazeutischen Verschlüssen mit einem abnehmbaren und verschwenkbaren Behandlungs- und Transportbehälter;
Fig. 2: eine teilweise geschnittene Seitenansicht eines Teils der Vorrichtung aus Fig. 1 bei abgenommenem Behandlungs- und Transportbehälter;
Fig. 3: eine vergrößerte Schnittansicht von zwei flüssigkeitsdicht miteinander verbundenen Anschlussstutzen eines Entleerungsanschlusses der Vorrichtung und eines Austrittsanschlusses in einer Zufuhr- bzw. Abfuhrleitung zwischen dem Behälter und der Vorrichtung;
Fig. 4a bis c: Vorderseitenansichten des von der Vorrichtung getragenen Behandlungs- und Transportbehälters in verschiedenen Schwenkstellungen;
Fig. 5: eine auseinandergezogene perspektivische Ansicht eines Schwenkantriebs der Vorrichtung.

Die in der Zeichnung dargestellte Vorrichtung 2 dient zur Reinigung, Sterilisation und ggf. zur Silikonisierung von Verschlüssen für pharmazeutische Verpackungsbehälter, die zu ihrer Behandlung in einen Behandlungs- und Transportbehälter 4 eingebracht und mit Hilfe der Vorrichtung 2 im Behälter 4 gereinigt, sterilisiert und ggf. silikonisiert werden, bevor sie im Behälter 4 zu einer Übergabestation (nicht dargestellt) einer Verpackungsmaschine transportiert werden, um sie dort nach einem Ankoppeln des Behälters 4 in sterilem Zustand zu entnehmen und mittels der Verpackungsmaschine einzeln auf den zuvor gefüllten pharmazeutischen Verpackungsbehältern anzubringen.

Wegen der hohen Anforderungen an die Reinheit erfolgen sowohl die Behandlung der Verschlüsse mittels der Reinigungs- und Sterilisationsvorrichtung 2 sowie der Transport des Behälters 4 mit den gereinigten und sterilisierten Verschlüssen und das Ankoppeln an die Übergabestation innerhalb eines Reinraums 6.

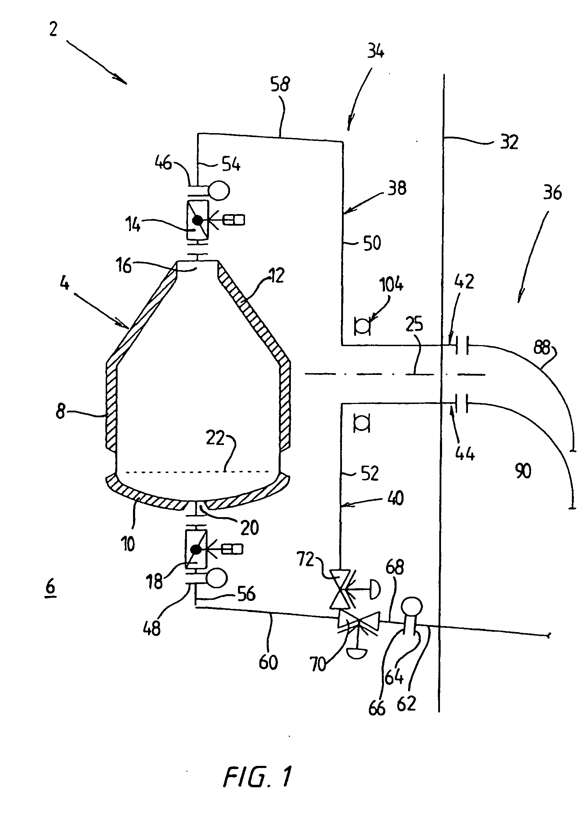

Wie am besten in Fig. 1 dargestellt, weist der Behandlungs- und Transportbehälter 4 in aufrechter Stellung ein im Wesentlichen zylindrisches Unterteil 8 mit einem konvex nach unten gewölbten Boden 10 und ein nach oben zu konisch verjüngtes Oberteil 12 auf. Am oberen Ende des Behälters 4 befindet sich eine durch ein Klappenventil 14 verschließbare Öffnung 16 mit größerem Öffnungsquerschnitt, durch welche die zu reinigenden und zu sterilisierenden Verschlüsse bei aufrechtem Behälter 4 in diesen eingefüllt werden und durch welche die gereinigten und sterilisierten Verschlüsse durch ihre Schwerkraft aus dem um 180 Grad gedreht an die Übergabestation angekoppelten umgekehrten Behälter 4 in die Übergabestation fallen. Am tiefsten Punkt des Bodens 10 befindet sich eine weitere durch ein Klappenventil 18 verschließbare Öffnung 20, durch die ebenso wie durch die Öffnung 16 Heißwasser, Kaltwasser, ein Wasser-Luft-Gemisch, Trocknungsluft, Dampf oder andere flüssige oder gasförmige Behandlungsmedien in den Behälter 4 zugeführt oder aus diesem abgeführt werden können. Im Inneren ist der Behälter 4 in einem geringen Abstand über dem Boden 10 mit einem siebförmigen Zwischenboden 22 versehen, der bei der Behandlung einen Hindurchtritt des Behandlungsmediums gestattet und bei aufrechtem Behälter 4 ein Abtropfen der Gegenstände erlaubt.

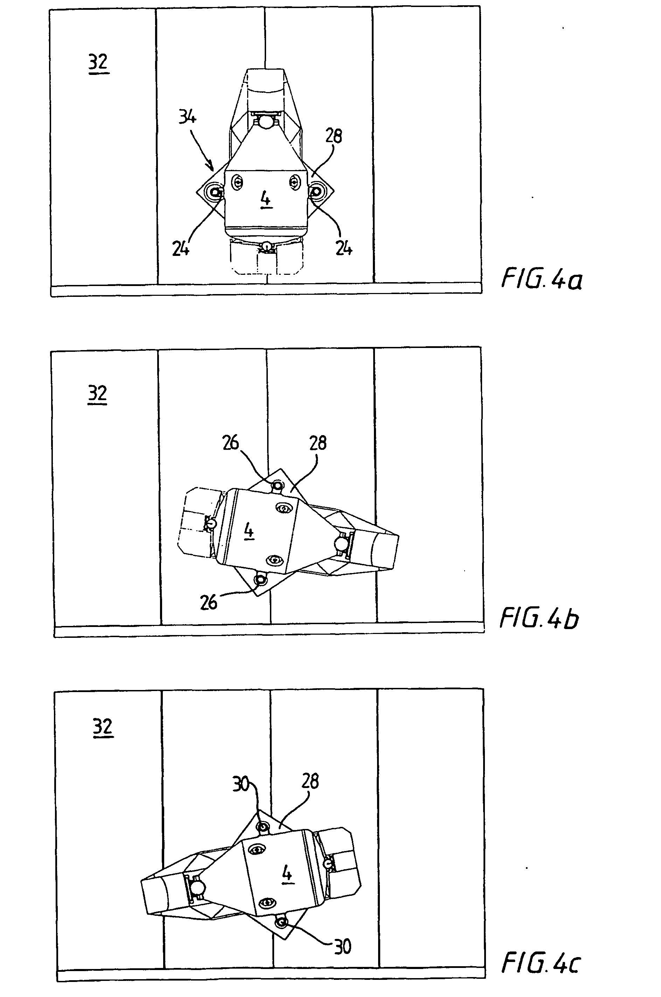

Wie am besten in den Figuren 4a bis 4c dargestellt, weist der Behälter 4 weiter zwei diametral entgegengesetzte seitliche Ausleger 24 mit zylindrischen Aufnahmebuchsen 26 auf, die bei aufrechtem Behälter 4 parallel und horizontal ausgerichtet sind. Diese Aufnahmebuchsen 26 dienen zur lösbaren Befestigung des Behälters 4 an einem schwenkbaren Tragelement 28 der Reinigungs- und Sterilisationsvorrichtung 2, das mit zwei überstehenden, in die Aufnahmebuchsen 26 einführbaren parallelen Tragdornen 30 versehen ist.

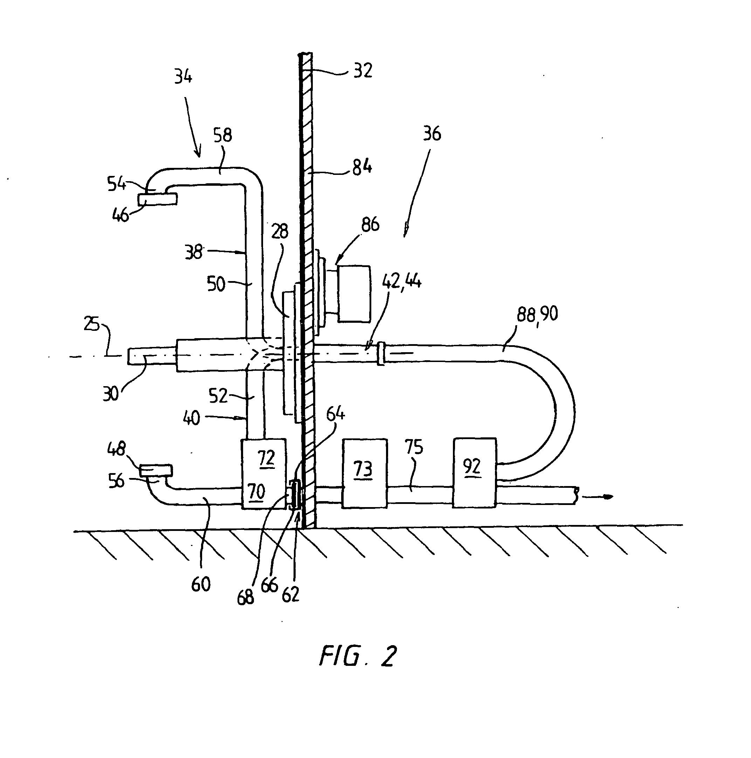

Die Reinigungs- und Sterilisationsvorrichtung 2 besteht im Wesentlichen aus einem in den Reinraum 6 überstehenden Reinraumteil 34 und einem durch eine Trennwand 32 vom Reinraumteil 34 getrennten Maschinenteil 36, wie am besten in Fig. 2 dargestellt.

Neben dem motorisch um eine horizontale Schwenkachse 25 schwenkbaren Tragelement 28 für den Behälter 4 umfasst der Reinraumteil 32 zwei in den Reinraum 6 überstehende Leitungsabschnitte 38, 40 einer Zufuhr- bzw. Abfuhrleitung 42, 44, durch die ein Behandlungsmedium wahlweise in den Behälter 4 zugeführt bzw. das aus dem Behälter 4 verdrängte Medium abgeführt werden kann. Jeder der beiden Leitungsabschnitte 38, 40 weist ein freies Ende mit einem Anschlussflansch 46, 48 auf, von denen sich nach dem Aufschieben des Behälters 4 auf die Tragdorne 30 des Tragelements 28 der eine mit einem komplementären Flansch des Ventils 14 an der oberen Behälteröffnung 16 und der andere mit einem komplementären Flansch des Ventils 18 an der unteren Behälteröffnung 20 flüssigkeits- und gasdicht verbinden lässt. Die beiden Leitungsabschnitte 38, 40 laufen im Bereich des schwenkbaren Tragelements 28 zusammen und sind durch das Tragelement 28 hindurch in den Maschinenteil 36 der Vorrichtung 2 geführt, wobei sie zusammen mit dem Tragelement 28 um dessen Schwenkachse 25 verschwenkbar sind. Die beiden Leitungsabschnitte 38, 40 bestehen jeweils aus zwei vertikalen Teilstücken 50, 54; 52, 56 und einem die vertikalen Teilstücke 50, 52; 54, 56 verbindenden Teilstück 58, 60, das zur Trennwand 32 hin leicht nach unten geneigt ist, wie am besten in Fig. 1 dargestellt.

Der Reinraumteil 34 umfasst darüber hinaus noch einen Entleerungsanschluss 62, der unterhalb des Tragelements 28 in der Trennwand 32 angeordnet ist und einen in den Reinraum 6 mündenden Anschlussflansch 64 aufweist. Der Anschluss 62 dient zur Restentleerung des Behälters 4 in dessen aufrechter Medienentleerstellung, in der sich der Anschlussflansch 64 flüssigkeits- und gasdicht mit einem komplementären Anschlussflansch 66 eines Austrittsanschlusses 68 in dem zur unteren Behälteröffnung 20 führenden Leitungsabschnitt 40 verbinden lässt. Der Entleerungsanschluss 62 liegt tiefer als die untere Behälteröffnung 20, so dass im Behälter 4 befindliches flüssiges Behandlungsmedium durch Schwerkraft vollständig in den Entleerungsanschluss 62 abläuft, wenn die beiden Anschlussflansche 64, 66 in der Medienentleerstellung des Behälters 4 miteinander verbunden und das Ventil 18 sowie ein vor dem Austrittsanschluss 68 angeordnetes erstes Wegeventil 70 geöffnet sind. Durch Öffnen eines zweiten Wegeventils 72 im vertikalen Teilstück 52 des Leitungsabschnitts 40 kann in diesem Teilstück 52 zurückgebliebenes flüssiges Behandlungsmedium ebenfalls durch den Entleerungsanschluss 62 in eine in den Maschinenteil 36 führende und hinter der Trennwand 32 durch ein Ventil 73 verschließbare Rohrleitung 75 abgeführt werden.

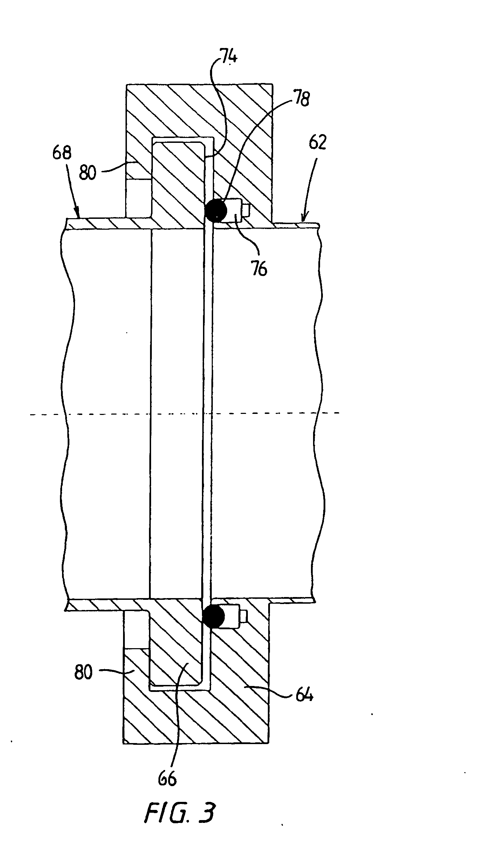

Zur Herstellung einer flüssigkeits- und gasdichten Verbindung zwischen den beiden Anschlussflanschen 64, 66 weist einer derselben, zweckmäßig der etwas über die Trennwand 32 überstehende Anschlussflansch 64 des Entleerungsanschlusses 62, gegenüber von einer ebenen ringförmigen radialen Dichtfläche 74 des anderen Anschlussflansches 66 eine ringförmige Aufnahmenut 76 auf, in die ein gummielastischer Dichtring 78 eingesetzt ist, wie in Fig. 3 dargestellt. Auf der von der Dichtfläche 74 abgewandten Stirnseite des Dichtrings 78 ist die Nut 76 mit Druckluft beaufschlagbar, um den Dichtring 78 gegen die Dichtfläche 74 anzupressen. Um ein Ausweichen des Anschlussflansches 66 in axialer Richtung infolge der vom Dichtring 78 ausgeübten Kraft zu vermeiden, ist der Flansch 64 an zwei diametral gegenüberliegenden Stellen auf seiner Ober- und Unterseite jeweils mit einem Vorsprung 80 versehen. Diese Vorsprünge 80 hintergreifen den Anschlussflansch 66 des Austrittsanschlusses 68, wenn die Durchtrittsöffnungen der beiden Anschlüsse 62, 68 miteinander fluchten, erlauben es jedoch, den Flansch 66 des Austrittsanschlusses 68 zusammen mit dem Behälter 4 und den Leitungsabschnitten 38, 40 um die Drehachse 25 des Tragelements 28 zu verschwenken.

Ähnliche, mit Druckluft beaufschlagbare Dichtringe sind auch in den Anschlussflanschen 46, 48 an den freien Enden der beiden Leitungsabschnitte 38, 40 angebracht, um diese nach dem Aufschieben des Behälters 4 auf die Tragdorne 30 des Tragelements 28 mit den benachbarten Flanschen an den Ventilen 14, 18 des Behälters 4 zu verbinden.

Der Maschinenteil 36 der Vorrichtung 2 kann unter anderem einen Heißwasserbereiter, einen Drucklufterzeuger und einen Dampferzeuger umfassen, die über die Zufuhr- bzw. Abfuhrleitung 42, 44 mit dem Behälter 4 verbindbar sind, jedoch keinen wesentlichen Teil der vorliegenden Erfindung bilden und daher zur Vereinfachung nicht dargestellt sind. Der Maschinenteil 36 der Vorrichtung 2 umfasst weiter einen Maschinenrahmen 84 zur Abstützung des Tragelements 28 und des Behälters 4, sowie einen Schwenkantrieb 86 zum motorischen Verschwenken der beiden Komponenten 4, 28 im Betrieb.

Wie am besten in Fig. 1 und 2 dargestellt, umfassen die Zufuhr- bzw. Abfuhrleitung 42, 44 auf der vom Reinraum 6 abgewandten Seite der Trennwand 34 zwei flexible Leitungsabschnitte 88, 90, die in der Medienentleerstellung des Behälters 4 in horizontaler Richtung beiderseits der Schwenkachse 25 angeordnet sind und nach hinten von der Trennwand 32 weg, im Bogen nach unten und wieder nach vorne auf die Trennwand 32 zu verlaufen. Am unteren Ende der beiden Leitungsabschnitte 88, 90 ist jeweils ein Wegeventil 92 angeordnet, das es gestattet, die Leitungsabschnitte wahlweise entweder mit dem Heißwasserbereiter, dem Drucklufterzeuger oder dem Dampferzeuger zu verbinden, oder alternativ mit einer tiefer gelegenen Entleerungsöffnung (nicht dargestellt), durch welche die gesamte Flüssigkeit aus den Leitungsabschnitten 88, 90 sowie den reinraumseitigen Leitungsstücken allein durch ihrer Schwerkraft aus den Leitungen 42, 44 abfließen kann. Die flexiblen Leitungsabschnitte 88, 90 weisen eine ausreichende Länge auf, so dass ihre reinraumseitigen Enden beim Verschwenken des Behälters 4 um einen Winkel von 130 Grad aus der aufrechten Stellung in beide Richtungen (vgl. Fig. 4a bis 4c) der Schwenkbewegung des Tragelements 28 folgen können, ohne dass es zum Auftreten von Zugspannungen in den Leitungsabschnitten 88, 90 kommt und diese nur minimal auf Torsion beansprucht werden.

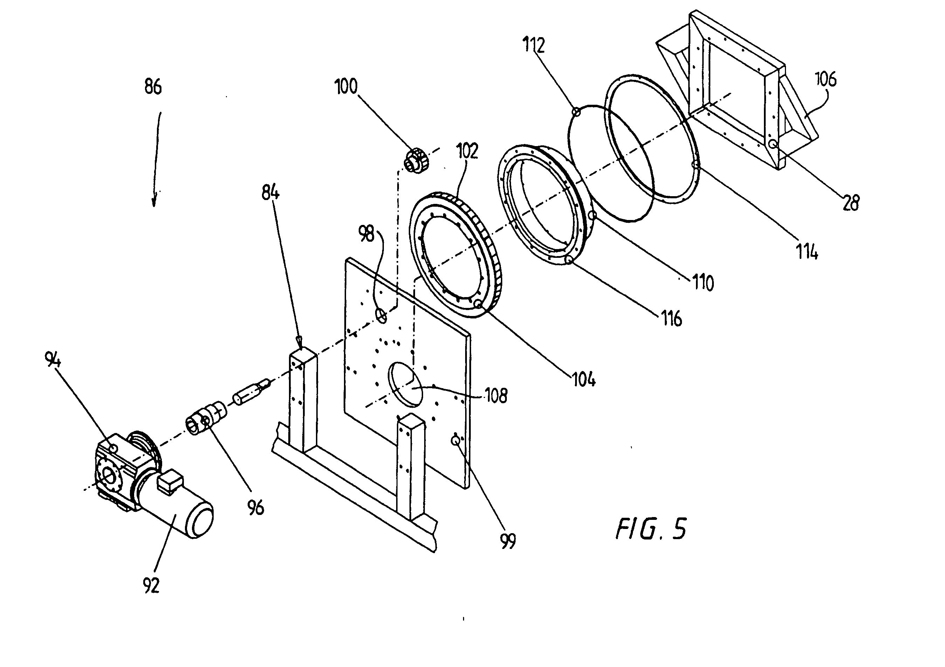

Der Schwenkantrieb 86 zum Verschwenken des Tragelements 28 und des Behälters 4 ist am besten in Fig. 5 dargestellt. Er besteht im Wesentlichen aus einem elektrischen Antriebsmotor 92 mit angeflanschtem Winkelgetriebe 94, dessen Abtriebswelle durch eine Steckwelle 96 verlängert ist. Die Steckwelle 96 durchsetzt eine Durchtrittsöffnung 98 in einer Halteplatte 99 und ein Antriebsritzel 100, das mit einem drehfest mit dem Tragelement 28 verbundenen Zahnkranz 102 im Zahneingriff steht. Der Zahnkranz 102 ist Teil des Außenrings eines zur Aufhängung des Tragelements 28 am Maschinenrahmen 84 dienenden Axial/Radiallagers 104, dessen Innenring durch die Halteplatte 99 hindurch am Maschinenrahmen 84 festgeschraubt ist. Das Tragelement 28 selbst besteht aus einem quadratischen Rahmen mit zwei seitlich überstehenden Auslegern 106, welche die Tragdorne 30 (in Fig. 5 nicht dargestellt) zur Aufhängung des Behälters 4 tragen.

Die Zufuhr- bzw. Abfuhrleitungen 42, 44 (in Fig. 5 ebenfalls nicht dargestellt) sind zusammen mit Druckluftleitungen und Stromleitungen durch das Axial-/Radiallager 104 und eine fluchtende Öffnung 108 in der Halteplatte 99 hindurch geführt. Zum Reinraum 6 hin ist diese Öffnung 108 durch eine kreisförmige Platte 110 verschlossen, die drehfest mit dem Tragelement 28 verbunden und mit eingeschweißten Leitungsdurchführungen (nicht dargestellt) versehen ist.

Zur Abdichtung der schwenkbaren Teile gegenüber den ortsfesten Teilen dient eine Dichtung 112, die von einem ortsfesten Haltering 114 gegen einen mit dem Zahnkranz 102 verschraubten Distanzring 116 gedrückt wird.

Die Leitungsdurchführungen der Platte 110, die einen Teil der Zufuhr- bzw. Abfuhrleitung 42, 44 bilden, weisen auf der vom Reinraum abgewandten Rückseite der Platte 110 jeweils einen Stutzen auf, mit dem das eine Ende jedes flexiblen Leitungsabschnitts 88, 90 verbunden ist. Die Stutzen sind so angebracht, dass sie divergieren, vorzugsweise unter einem Winkel von 90 Grad, und in der Medienentleerstellung des Behälters 4 horizontal ausgerichtet oder leicht nach unten geneigt sind.

Im Betrieb wird der aufrechte Behälter 4 im Reinraum 6 an eine Füllstation (nicht dargestellt) angekoppelt, wo er zuerst durch seine obere Öffnung 16 mit den zu behandelnden Verschlüssen befüllt wird. Nach dem Schließen des Ventils 14 und dem Abnehmen des Behälters 4 wird dieser zur Reinigungs- und Sterilisationsvorrichtung 2 gebracht, wo er an den Tragdornen 30 des Tragelements 28 aufgehängt wird und die Flansche der Ventile 14, 18 mit den benachbarten Anschlussflanschen 46, 48 der Leitungsabschnitte 38, 40 gekuppelt werden. Nach dem Öffnen der Ventile 14, 18 kann der Behälter 4 zum Beispiel durch die Leitung 42 hindurch aus dem Maschinenteil 36 mit Heißwasser oder einem Wasser-Luft-Gemisch beschickt werden, um die Verschlüsse zu reinigen und anhaftende Fremdkörper abzuspülen.

Gleichzeitig kann der Behälter 4 zusammen mit dem Tragelement 28 um die horizontale Drehachse 25 des Lagers 104 verschwenkt werden, um die Reinigungswirkung zu verbessern und insbesondere bei der Reinigung von Verschlüssen aus Gummi ein Verklumpen zu verhindern. Das Wasser oder Wasser-Luft-Gemisch kann durch den Behälter 4 hindurch zu dessen oberer Öffnung 16 und von dort durch die andere Leitung 44 zurück in den Maschinenteil 36 strömen. Dabei kann die Strömungsrichtung nach Wunsch auch umgekehrt werden.

Um den Behälter 4 und die Leitungen 42, 44 vor einer anschließenden Sterilisation der Verschlüsse vollständig vom Restwasser zu entleeren, wird zuerst der Behälter 4 in seine aufrechte Medienentleerstellung gebracht. In dieser Stellung wird der Anschlussflansch 66 des Austrittsschlusses 68 durch Beaufschlagung des Dichtrings 78 mit Druckluft mit dem Anschlussflansch 64 des Entleerungsanschlusses 62 verbunden und dann das Wegeventil 70 geöffnet, so dass das noch im Behälter 4 befindliche Wasser allein durch seine Schwerkraft vollständig in den Entleerungsanschluss 62 strömt und dabei eventuell zwischen dem Zwischenboden 22 und dem Boden 10 zurück gebliebene abgereinigte Partikel mit sich führt. Anschließend wird das Wegeventil 72 geöffnet, um das im Teilstück 52 des Leitungsabschnitts 40 befindliche Wasser durch die Anschlüsse 62, 68 zu entleeren.

Anschließend wird das Wegeventil 70 geschlossen, um wie beim Spülen einen Austritt von Behandlungsmedium durch den Austrittsanschluss 68 zu verhindern, bevor der Behälter 4 zur Sterilisation der im Behälter 4 befindlichen Verschlüsse erneut in eine Schwenkbewegung versetzt und durch die Leitung 42 mit Sattdampf gespeist wird, wobei der Sattdampf nach seinem Hindurchtritt durch den Behälter 4 durch die Leitung 44 in den Maschinenteil 36 zurückgeführt wird. Alternativ ist es auch möglich, dem Behälter 4 im Stillstand Sattdampf durchzuführen, wobei in diesem Fall der Austrittsanschluss 68 mit dem Entleerungsanschluss 62 verbunden und die Wegeventil 70, 72 so geschaltet sind, dass Kondensat durch den Entleerungsanschluss 62 abfließen kann.

## Patentansprüche

1. Vorrichtung zur Behandlung von kleineren Gegenständen, insbesondere zur Reinigung und Sterilisation von Spritzenkolben oder Verschlüssen von Spritzen, Infusionsflaschen, Vials für pharmazeutische Zwecke, mit einem Behandlungs- und Transportbehälter (4) zur Aufnahme einer Mehrzahl der Gegenstände, der an einem motorisch schwenkbaren Tragelement (28) der Vorrichtung lösbar befestigbar ist und zwei verschließbare Öffnungen (16,20) aufweist, die nach der Befestigung des Behandlungs- und Transportbehälters (4) am Tragelement (28) beiderseits von dessen horizontaler Schwenkachse (25) angeordnet und jeweils mit einer zum Teil entlang der Schwenkachse (25) verlaufenden Zufuhr- bzw. Abfuhrleitung (42, 44) der Vorrichtung für ein flüssiges Behandlungsmedium verbindbar sind, **dadurch gekennzeichnet, dass** die Vorrichtung (2) einen ortsfesten Entleerungsanschluss (62) für das flüssige Behandlungsmedium aufweist, wobei der Entleerungsanschluss (62) tiefer liegt als diejenige Behälteröffnung (20), die in einer Medienentleerstellung des Behandlungs- und Transportbehälters (4) nach unten weist, und wobei der Entleerungsanschluss (62) in der Medienentleerstellung lösbar mit der nach unten weisenden Öffnung (20) verbindbar ist.

2. Vorrichtung nach Anspruch 1, **dadurch gekennzeichnet, dass** der Entleerungsanschluss (62) über ein um die Schwenkachse (25) verschwenkbares Teilstück (60) der Zufuhr- bzw. Abfuhrleitung (44) mit der Öffnung (20) verbindbar ist.

3. Vorrichtung nach Anspruch 2, **dadurch gekennzeichnet, dass** das zwischen der Öffnung (20) und dem Entleerungsanschluss (62) angeordnetes Teilstück (60) der Zufuhr- bzw. Abfuhrleitung (44) zum Entleerungsanschluss (62) hin geneigt ist.

4. Vorrichtung nach Anspruch 2 oder 3, **dadurch gekennzeichnet, dass** das mit der Öffnung (20) verbundene Teilstück (60) der Zufuhr- bzw. Abfuhrleitung (44) mindestens ein Ventil (70) aufweist.

5. Vorrichtung nach einem der Ansprüche 2 bis 4, **dadurch gekennzeichnet, dass** der Entleerungsanschluss (62) einen Anschlussflansch (64) aufweist, der in der Medienentleerstellung des Behandlungs- und Transportbehälters (4) einem Anschlussflansch (66) in der Zufuhr- bzw. Abfuhrleitung (44) in geringem Abstand gegenüberliegt.

6. Vorrichtung nach Anspruch 5, **dadurch gekennzeichnet, dass** einer der beiden Anschlussflansche (64, 66) mit einem axial verschiebbaren und gegen eine Dichtfläche des jeweils anderen Flansches (66, 64) anpressbaren Dichtring (78) versehen ist.

7. Vorrichtung nach einem der Ansprüche 2 bis 6, **dadurch gekennzeichnet, dass** einer der beiden Anschlussflansche (64, 66) den jeweils anderen Anschlussflansch (66, 64) hintergreift, jedoch ein Verschwenken des einen Flansches (66) in Bezug zum anderen (64) um die Schwenkachse des Tragelements (28) nicht behindert.

8. Vorrichtung nach einem der vorangehenden Ansprüche, **dadurch gekennzeichnet, dass** in einer Medienentleerstellung des Behandlungs- und Transportbehälters (4) die beiden als Zufuhr- bzw. Abfuhrleitung dienenden Leitungen (42, 44) nach einer Zusammenführung im Bereich des Tragelements (28) auf dessen vom Behandlungs- und Transportbehälter (4) abgewandter Seite bis in den Bereich einer verschließbaren Entleerungsöffnung ausschließlich abwärts oder über kurze Strecken horizontal verlaufen.

9. Vorrichtung nach Anspruch 8, **dadurch gekennzeichnet, dass** die Zufuhr- bzw. Abfuhrleitungen (42, 44) auf der vom Behandlungs- und Transportbehälter (4) abgewandten Seite des Tragelements (28) jeweils ein flexibles Leitungsstück (88, 90) umfassen, das in der Medienentleerstellung des Behandlungs- und Transportbehälters (4) um einen Winkel zwischen 90 und 180 Grad nach unten und in Richtung des Transportbehälters (4) gebogen ist.

10. Verfahren zur Behandlung von kleineren Gegenständen, insbesondere zur Reinigung und Sterilisation von Spritzenkolben oder Verschlüssen von Spritzen, Infusionsflaschen, Vials für pharmazeutische Zwecke, bei dem eine Mehrzahl der Gegenstände in einen Behandlungs- und Transportbehälter eingebracht wird, der zwei verschließbare Öffnungen aufweist, bei dem der Behandlungs- und Transportbehälter anschließend lösbar an einem motorisch schwenkbaren Tragelement einer Behandlungsvorrichtung befestigt wird, so dass die Öffnungen beiderseits von einer horizontalen Schwenkachse des Tragelements angeordnet sind, und bei dem die Öffnungen jeweils mit einer entlang der Schwenkachse durch das Tragelement verlaufenden Zufuhr- bzw. Abfuhrleitung für ein flüssiges Behandlungsmedium verbunden werden, **dadurch gekennzeichnet, dass** die in einer Medienentleerstellung des Behandlungs- und Transportbehälters (4) nach unten weisende Öffnung (20) über ein um die Schwenkachse (25) verschwenkbares Teilstück der Zufuhr- bzw. Abfuhrleitung (44) lösbar mit einem tiefer liegenden ortsfesten Entleerungsanschluss (62) für das flüssige Behandlungsmedium verbunden wird, und dass das flüssige Behandlungsmedium durch den Entleerungsanschluss (62) vollständig abgeführt wird.

## Claims

1. Device for treating small objects, in particular for cleaning and sterilizing syringe plungers or the stoppers of syringes, infusion bottles, or vials for pharmaceutical purposes, with a treatment and transport container (4) for receiving a multiplicity of the objects, said treatment and transport container (4) being able to be secured releasably on a support element (28) of the device, which support element (28) can be pivoted by motor, and comprising two closable openings (16, 20) which, after the treatment and transport container (4) has been secured on the support element (28), are arranged on both sides of the horizontal pivot axis (25) thereof and can each be connected to an inlet and outlet line (42, 44) which partly extends along the pivot axis (25) and carries a liquid treatment medium, **characterized in that** the device (2) has a positionally fixed discharge piece (62) for the liquid treatment medium, the discharge piece (62) lying lower down than the container opening (20) directed downwards when the treatment and transport container (4) is in a media discharge position, and the discharge piece (62) being able to be connected releasably to the downwardly directed opening (20) in the media discharge position.

2. Device according to Claim 1, **characterized in that** the discharge piece (62) can be connected to the opening (20) via a partial section (60) of the inlet and outlet line (44) that can be pivoted about the pivot axis (25).

3. Device according to Claim 2, **characterized in that** the partial section (60) of the inlet and outlet line (44) arranged between the opening (20) and the discharge piece (62) is inclined towards the discharge piece (62).

4. Device according to Claim 2 or 3, **characterized in that** the partial section (60) of the inlet and outlet line (44) connected to the opening (20) has at least one valve (70).

5. Device according to one of Claims 2 to 4, **characterized in that** the discharge piece (62) has a connection flange (64) which, when the treatment and transport container (4) is in the media discharge position, lies a short distance opposite a connection flange (66) in the inlet and outlet line (44).

6. Device according to Claim 5, **characterized in that** one of the two connection flanges (64, 66) is provided with an axially displaceable sealing ring (78) which can be pressed against a sealing face of the respective other flange (66, 64).

7. Device according to one of Claims 2 to 6, **characterized in that** one of the two connection flanges (64, 66) engages behind the respective other connection flange (66, 64), but does not impede pivoting of one flange (66) relative to the other (64) about the pivot axis of the support element (28).

8. Device according to one of the preceding claims, **characterized in that**, when the treatment and transport container (4) is in a media discharge position, the two lines (42, 44) serving as inlet and outlet line, after coming together in the area of the support element (28), extend exclusively downwards, or over short distances horizontally, on the side of the support element (28) directed away from the treatment and transport container (4), into the area of a closable discharge opening.

9. Device according to Claim 8, **characterized in that** the inlet and outlet lines (42, 44), on the side of the support element (28) directed away from the treatment and transport container (4), each comprise a flexible line section (88, 90) which, with the treatment and transport container (4) in the media discharge position, is bent downwards at an angle of between 90 and 180 degrees and in the direction of the transport container (4).

10. Method for treating small objects, in particular for cleaning and sterilizing syringe plungers or the stoppers of syringes, infusion bottles, or vials for pharmaceutical purposes, in which method a multiplicity of the objects is introduced into a treatment and transport container comprising two closable openings, the treatment and transport container is then secured releasably on a support element of a treatment device, which support element can be pivoted by motor, so that the openings are arranged on both sides of a horizontal pivot axis of the support element, and the openings are each connected to an inlet and outlet line which extends along the pivot axis through the support element and carries a liquid treatment medium, **characterized in that** the opening (20), directed downwards in a media discharge position of the treatment and transport container (4), is releasably connected, via a partial section of the inlet and outlet line (44) pivotable about the pivot axis (25), to a lower-lying, positionally fixed discharge piece (62) for the liquid treatment medium, and **in that** the liquid treatment medium is drained off completely through the discharge piece (62).

## Revendications

1. Dispositif pour traiter de petits objets, notamment pour le nettoyage et la stérilisation de pistons d'injection ou de fermoirs à seringue, flacons de perfusion, flacons à usage pharmaceutique, avec un récipient de traitement et de transport (4) destiné à recevoir une pluralité d'objets, lequel peut être fixé de manière amovible à un élément porteur à pivotement motorisé (28) du dispositif et présente deux ouvertures (16, 20) fermables qui, après la fixation du récipient de traitement et de transport (4) à l'élément porteur (28), sont disposées des deux côtés de son axe de pivotement horizontal (25) et peuvent à chaque fois être reliées avec une conduite d'arrivée ou d'évacuation (42, 44) du dispositif pour un fluide de traitement liquide qui s'étend en partie le long de l'axe de pivotement (25), **caractérisé en ce que** le dispositif (2) présente un raccord de vidange fixe (62) pour le fluide de traitement liquide, le raccord de vidange (62) étant plus bas que l'ouverture de récipient (20) qui est dirigée vers le bas dans une position de vidange du fluide du récipient de traitement et de transport (4), et le raccord de vidange (62) pouvant être relié de manière amovible avec l'ouverture (20) dirigée vers le bas dans une position de vidange du fluide.

2. Dispositif selon la revendication 1, **caractérisé en ce que** le raccord de vidange (62) peut être relié avec l'ouverture (20) par le biais d'une partie (60) de la conduite d'arrivée et d'évacuation (44) qui peut pivoter autour de l'axe de pivotement (25).

3. Dispositif selon la revendication 2, **caractérisé en ce que** la partie (60) de la conduite d'arrivée ou d'évacuation (44) disposée entre l'ouverture (20) et le raccord de vidange (62) est inclinée en direction du raccord de vidange (62).

4. Dispositif selon la revendication 2 ou 3, **caractérisé en ce que** la partie (60) de la conduite d'arrivée ou d'évacuation (44) reliée avec l'ouverture (20) présente au moins une vanne (70).

5. Dispositif selon l'une des revendications 2 à 4, **caractérisé en ce que** le raccord de vidange (62) présente une bride de raccordement (64) qui, lorsque le récipient de traitement et de transport (4) est en position de vidage du fluide, se trouve à une faible distance à l'opposé d'une bride de raccordement (66) dans la conduite d'arrivée ou d'évacuation (44).

6. Dispositif selon la revendication 5, **caractérisé en ce que** l'une des deux brides de raccordement (64, 66) est munie d'une bague d'étanchéité (78) pouvant coulisser dans le sens axial et pouvant être comprimée contre une surface d'étanchéité à chaque fois de l'autre bride (66, 64).

7. Dispositif selon l'une des revendications 2 à 6, **caractérisé en ce que** l'une des deux brides de raccordement (64, 66) vient en prise par l'arrière à chaque fois avec l'autre bride de raccordement (66, 64), mais n'empêche cependant pas un pivotement de cette bride (66) par rapport à l'autre (64) autour de l'axe de pivotement de l'élément porteur (28).

8. Dispositif selon l'une des revendications précédentes, **caractérisé en ce que** dans une position de vidage du fluide du récipient de traitement et de transport (4), les deux conduites (42, 44) faisant office de conduite d'arrivée ou d'évacuation s'étendent exclusivement vers le bas ou, par courtes sections, dans le sens horizontal vers un regroupement dans la zone de l'élément porteur (28) sur son côté opposé au récipient de traitement et de transport (4) jusque dans la zone d'une ouverture de vidange fermable.

9. Dispositif selon la revendication 8, **caractérisé en ce que** les conduites d'arrivée ou d'évacuation (42, 44), sur le côté de l'élément porteur (28) à l'opposé du récipient de traitement et de transport (4), comprennent à chaque fois une partie de conduite souple (88, 90) qui, en position de vidange du fluide du récipient de traitement et de transport (4), est coudée selon un angle compris entre 90 et 180 degrés vers le bas en direction du récipient de transport (4).

10. Procédé pour traiter de petits objets, notamment pour le nettoyage et la stérilisation de pistons d'injection ou de fermoirs à seringue, flacons de perfusion, flacons à usage pharmaceutique, avec lequel une pluralité d'objets est introduite dans un récipient de traitement et de transport, lequel présente deux ouvertures fermables, avec lequel le récipient de traitement et de transport est ensuite fixé de manière amovible à un élément porteur à pivotement motorisé d'un dispositif de traitement de manière à ce que les ouvertures soient disposées des deux côtés d'un axe de pivotement horizontal de l'élément porteur, et avec lequel les ouvertures sont à chaque fois reliées avec une conduite d'arrivée ou d'évacuation pour un fluide de traitement liquide qui s'étend le long de l'axe de pivotement à travers l'élément porteur, **caractérisé en ce que** l'ouverture (20) qui est dirigée vers le bas dans une position de vidange du fluide du récipient de traitement et de transport (4) est reliée de manière amovible par le biais d'une partie de la conduite d'arrivée ou d'évacuation (44) qui peut pivoter autour de l'axe de pivotement (25) à un raccord de vidange (62) Fixe pour le fluide de traitement liquide qui se trouve plus bas et que le fluide de traitement liquide est totalement évacué à travers le raccord de vidange (62).
